(19) Europäisches Patentamt

European Patent Office

Office européen des brevets

(11) EP 2 513 026 B1

(12) FASCICULE DE BREVET EUROPEEN

(45) Date de publication et mention de la délivrance du brevet:
**12.11.2014 Bulletin 2014/46**

(21) Numéro de dépôt: **10805717.5**

(22) Date de dépôt: **10.12.2010**

(51) Int Cl.:
*C07C 45/52* [(2006.01)]    *C07C 51/25* [(2006.01)]
*C07C 47/22* [(2006.01)]    *C07C 57/04* [(2006.01)]

(86) Numéro de dépôt international:
**PCT/FR2010/052668**

(87) Numéro de publication internationale:
**WO 2011/073552 (23.06.2011 Gazette 2011/25)**

(54) **PROCEDE DE FABRICATION D'ACROLEINE ET/OU D'ACIDE ACRYLIQUE A PARTIR DE GLYCEROL**

VERFAHREN ZUR HERSTELLUNG VON ACROLEIN UND/ODER ACRYLSÄURE AUS GLYCEROL

METHOD FOR MANUFACTURING ACROLEIN AND/OR ACRYLIC ACID FROM GLYCEROL

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **14.12.2009 FR 0958947**
**16.03.2010 US 314237 P**

(43) Date de publication de la demande:
**24.10.2012 Bulletin 2012/43**

(73) Titulaire: **Arkema France**
**92700 Colombes (FR)**

(72) Inventeurs:
• **DEVAUX, Jean-François**
**F-69510 Soucieu En Jarrest (FR)**
• **LOZOWSKI, André**
**F-64230 Lescar (FR)**
• **TLILI, Nabil**
**68100 Mulhouse (FR)**

(74) Mandataire: **Bonnel, Claudine et al**
**ARKEMA FRANCE**
**Département Propriété Industrielle**
**420, rue d'Estienne d'Orves**
**92705 Colombes Cedex (FR)**

(56) Documents cités:
**WO-A1-2010/066513    WO-A2-2006/092272
WO-A2-2008/087315**

**Description**

Domaine de l'invention

La présente invention concerne la fabrication d'acroléine et d'acide acrylique bioressourcés à partir de glycérol comme matière première et s'inscrit plus particulièrement dans le cadre d'un procédé de fabrication d'acroléine et d'acide acrylique suivant lequel on effectue la réaction de déshydratation de glycérol en acroléine et on élimine les composés organiques plus lourds que l'eau, susceptibles d'être présents dans les différents flux du procédé, de façon à obtenir un flux recyclable à l'étape de déshydratation sans accumulation d'impuretés lourdes, tout en minimisant la consommation d'eau et le rejet de flux aqueux pollués.

Technique antérieure

Le procédé de synthèse de l'acide acrylique le plus largement exploité industriellement utilise une réaction catalytique du propylène à l'aide d'un mélange contenant de l'oxygène. Cette réaction est conduite généralement en phase vapeur, et le plus souvent en deux étapes : la première étape réalise l'oxydation sensiblement quantitative du propylène en un mélange riche en acroléine, dans lequel l'acide acrylique est minoritaire, puis la deuxième étape réalise l'oxydation sélective de l'acroléine en acide acrylique. Les conditions de réaction de ces deux étapes, réalisées dans deux réacteurs en série ou dans les 2 zones de réaction d'un seul réacteur « single », sont différentes et nécessitent des catalyseurs adaptés à chacune des réactions.

Depuis quelques années, les industriels conduisent des travaux de recherche et de développement sur des procédés de synthèse de l'acroléine et de l'acide acrylique utilisant des matières premières bioressourcées. A l'origine de ces travaux est le souci d'éviter d'utiliser à l'avenir les matières premières fossiles, tel que le propylène dont l'origine pétrolière contribue au réchauffement climatique dû à l'effet de serre. Par ailleurs, son coût ne peut à l'avenir qu'augmenter avec la diminution des réserves pétrolières mondiales.

Parmi ces procédés alternatifs à partir de matières premières non fossiles, on peut citer les procédés utilisant comme matière première l'acide 3-hydroxypropionique obtenu par fermentation de glucose ou de mélasse provenant de la biomasse.

On peut citer également les procédés à partir de glycérol (appelé aussi glycérine), issu de la méthanolyse des huiles végétales ou animales en même temps que les esters méthyliques qui sont, eux, employés notamment comme carburants ou combustibles dans le gazole et le fioul domestique. Ce glycérol est un produit naturel qui jouit d'une aura "verte", il est disponible en grande quantité et peut être stocké et transporté sans difficulté. La méthanolyse des huiles végétales ou des graisses animales peut s'effectuer selon différents procédés bien connus, notamment en utilisant une catalyse homogène telle que la soude ou le méthylate de sodium en solution dans le méthanol, ou en utilisant une catalyse hétérogène. On pourra se reporter sur ce sujet à l'article de D. Ballerini et al. dans l'Actualité Chimique de novembre-décembre 2002.

Les procédés utilisant l'acide 3-hydroxypropionique comme matière première ont un inconvénient majeur du point de vue économique. Ils mettent en jeu une réaction de fermentation qui est réalisée nécessairement en condition très diluée dans l'eau. Pour obtenir l'acide acrylique, une quantité très importante d'eau doit être éliminée par distillation, au prix d'un coût énergétique très important. Par ailleurs, l'énergie dépensée pour séparer l'eau, énergie produite à partir de matière fossile, vient dégrader fortement l'avantage initial de produire l'acide acrylique à partir de cette matière première bioressourcée. On peut citer dans ce domaine la demande WO2006/092271 qui décrit un procédé de production de polymères à partir d'acide acrylique préparé par voie enzymatique, notamment à partir de carbohydrate.

Le glycérol est reconnu aujourd'hui comme matière première adaptée pour envisager la fabrication industrielle de l'acroléine et de l'acide acrylique bioressourcés.

La réaction mise en jeu pour obtenir l'acroléine à partir du glycérol est :

$$CH_2OH\text{-}CHOH\text{-}CH_2OH \rightarrow CH_2\text{=}CH\text{-}CHO + 2H_2O$$

Cette étape est suivie d'une oxydation de l'acroléine pour obtenir l'acide acrylique.

Différents procédés de synthèse de l'acroléine à partir du glycérol sont décrits dans la littérature. On peut citer les documents FR 695931 ; US 2,558,520 ; WO 99/05085 ; US 5,387,720 ; WO 06/087083 ; WO 06/087084 ; WO 09/044081.

Dans la demande de brevet EP 1 710 227, le produit de réaction résultant de la réaction de déshydratation du glycérol en phase gaz, est soumis à une étape ultérieure d'oxydation en phase gaz pour obtenir de l'acide acrylique. Le procédé est mis en oeuvre dans deux réacteurs en série, comportant chacun un catalyseur adapté à la réaction mise en oeuvre. Il est préconisé d'ajouter de l'oxygène au mélange gazeux alimentant le second réacteur, afin d'améliorer la réaction d'oxydation et obtenir l'acide acrylique avec un rendement élevé. Ce procédé en deux étapes est mis en oeuvre avec du glycérol pur ou avec des solutions aqueuses comportant plus de 50 % en poids de glycérol. Il est conseillé d'utiliser une solution de glycérol concentrée afin de limiter le coût énergétique lié à l'évaporation de la solution aqueuse et le coût lié au traitement des eaux résiduaires. Cependant, si la concentration en glycérol est trop élevée, il risque de se produire davantage de réactions parasites conduisant à de nombreux sous-produits, comme la formation d'éthers de glycérol, ou des réactions entre l'acroléine ou l'acide acrylique produits et le glycérol. Ces sous-produits lourds ont tendance à rester sur le catalyseur de déshydratation et conduisent au cokage du catalyseur et à sa désactivation très rapide.

La demande WO 06/136336 décrit un procédé de synthèse d'acroléine et d'acide acrylique dans lequel la réaction de déshydratation est suivie d'une étape de séparation en une phase riche en acroléine et une phase appauvrie en acroléine, cette dernière phase, riche en eau, étant renvoyée en amont du réacteur de déshydratation afin de diluer le glycérol et obtenir une phase aqueuse comportant moins de 10% en glycérol.

Dans cette demande WO 06/136336 qui a trait essentiellement à un procédé de déshydratation en phase liquide, la phase appauvrie en acroléine et riche en eau contient aussi des composés plus lourds formés pendant la réaction de déshydratation qui ont tendance à former dans l'étape de réaction des composés lourds qui encrassent le catalyseur et provoquent sa désactivation.

La demande internationale WO 2006/092272 décrit un procédé de préparation d'acide acrylique à partir de glycérol comportant soit une étape de déshydratation du glycérol en phase liquide, soit une étape de déshydratation en phase gaz. Le mélange réactionnel contenant l'acroléine obtenue à partir de la réaction de déshydratation du glycérol est mis en contact avec de l'eau dans une unité de quench avant d'être adressé au réacteur d'oxydation. En présence d'un flux important d'eau, le catalyseur d'oxydation de l'acroléine risque de perdre rapidement son efficacité et sa tenue mécanique rendant la maintenance d'un tel procédé difficile. Selon la figure 5 de ce document, le mélange réactionnel issu de la déshydratation en phase liquide, est soumis à une distillation séparant, d'une part les produits légers de point d'ébullition inférieur à celui de l'acroléine, d'autre part une fraction comportant les produits lourds de point d'ébullition supérieur à celui de l'acroléine, cette seconde fraction, riche en eau, étant renvoyée à l'étape de réaction après avoir éliminé les impuretés dans un séparateur équipé d'une membrane. Le principe d'un tel recyclage peut néanmoins conduire à l'accumulation de certaines impuretés dans la boucle d'eau ainsi générée, en raison du manque de sélectivité de la membrane ou de son encrassement.

La demande WO 08/087315 décrit un procédé de préparation d'acide acrylique à partir de glycérol en deux étapes, dans lequel on met en oeuvre une étape intermédiaire consistant à condenser au moins en partie l'eau et les sous-produits lourds présents dans le flux issu de la première étape de déshydratation, avant d'adresser le flux au réacteur d'oxydation. Ce procédé permet l'utilisation de solutions aqueuses diluées de glycérol, produisant un effet bénéfique sur la réaction de déshydratation, tout en limitant une dégradation possible du catalyseur d'oxydation d'acroléine en présence d'une trop grande quantité d'eau. Le flux aqueux ainsi généré par l'étape de condensation est envoyé, en tout ou partie, soit vers une colonne de rectification pour récupérer les produits légers éventuellement présents, soit vers une station de traitement des eaux usées présentant cependant l'inconvénient de traitements coûteux avant rejet dans le milieu naturel de quantités importantes d'effluents aqueux. Alternativement, ce flux peut être envoyé vers un oxydeur thermique où il est incinéré, ou encore une partie de ce flux peut être recyclée directement pour diluer le glycérol à la concentration souhaitée, auquel cas, il peut y avoir accumulation d'impuretés dans la boucle d'eau ainsi formée et risque de cokage du catalyseur de déshydratation.

La présente invention se propose de remédier aux inconvénients que présentent les procédés de fabrication d'acide acrylique sus-mentionnés, afin d'améliorer significativement le procédé de fabrication de l'acide acrylique comportant une première étape de déshydratation du glycérol en acroléine, suivie d'une étape d'oxydation de l'acroléine en acide acrylique, sur les points suivants :

- réduction de la consommation d'eau tout en assurant une optimisation de la réaction de déshydratation du glycérol en présence d'eau,
- limitation de rejets aqueux pollués,
- réduction de la consommation énergétique et de la taille des équipements,
- limitation des pertes de produit tout en assurant une récupération efficace des produits de réaction,
- augmentation de la durée de cycle des catalyseurs de déshydratation et d'oxydation.

A cet effet, il est proposé de séparer les sous-produits lourds, notamment les composés organiques plus lourds que l'eau, susceptibles d'être présents dans les différents flux du procédé, de façon à obtenir un flux recyclable à l'étape de déshydratation exempt le plus possible de ces impuretés lourdes. La séparation des impuretés lourdes est réalisée par différence de points d'ébullition et peut être mise en oeuvre à différents endroits du procédé, ce qui permet de générer une boucle d'eau fermée dans le procédé sans accumulation d'impuretés lourdes et avec une optimisation énergétique du système.

Résumé de l'invention

La présente invention a donc pour objet un procédé de fabrication d'acroléine à partir de glycérol comprenant au moins les étapes suivantes :

a) on soumet du glycérol à une réaction de déshydratation pour obtenir un flux aqueux contenant de l'acroléine,
b) on sépare le flux issu de l'étape a) en une phase riche en acroléine et une phase aqueuse appauvrie en acroléine, dans des conditions telles que 20% à 95% massiques de l'eau présente dans le flux issu de l'étape a) sont éliminés dans la phase appauvrie en acroléine,
c) on recycle tout ou partie de la phase aqueuse appauvrie en acroléine à l'étape a), caractérisé en ce que l'on met en oeuvre une étape de séparation de composés organiques plus lourds que l'eau par différence de point d'ébullition, appliquée à l'un au moins des flux suivants : le flux entrant à l'étape a), le flux aqueux sortant de l'étape a), la phase aqueuse appauvrie en acroléine de l'étape b).

L'invention a aussi pour objet un procédé de fabrication d'acide acrylique à partir de glycérol comprenant au moins les étapes suivantes :

a) on soumet du glycérol à une réaction de déshydratation pour obtenir un flux aqueux contenant de l'acroléine,
b) on sépare le flux issu de l'étape a) en une phase riche en acroléine et une phase aqueuse appauvrie en acroléine, dans des conditions telles que 20% à 95% massiques de l'eau présente dans le flux issu de l'étape a) sont éliminés dans la phase appauvrie en acroléine,
c) on recycle tout ou partie de la phase aqueuse appauvrie en acroléine à l'étape a),
d) on soumet la phase riche en acroléine à une réaction d'oxydation catalytique pour obtenir un flux contenant de l'acide acrylique,
e) on soumet le flux issu de l'étape d) à un ou plusieurs traitements de purification, et l'on récupère l'acide acrylique purifié,
caractérisé en ce que l'on met en oeuvre une étape de séparation de composés organiques plus lourds que l'eau, par différence de point d'ébullition, appliquée à l'un au moins des flux suivants : le flux entrant à l'étape a), le flux aqueux sortant de l'étape a), la phase aqueuse appauvrie en acroléine de l'étape b).

Dans un mode de réalisation préféré de l'invention, on applique une étape de séparation des sous-produits lourds par différence de point d'ébullition au flux entrant à l'étape a) et/ou à la phase aqueuse appauvrie en acroléine de l'étape b), associée ou non à une étape de séparation de sous-produits lourds par différence de point d'ébullition appliquée au flux aqueux sortant de l'étape a).

Dans la suite de l'exposé de l'invention, les expressions « sous-produits lourds », « composés lourds » et « composés organiques plus lourds que l'eau » ont la même signification.

D'autres caractéristiques et avantages de l'invention ressortiront mieux à la lecture de la description détaillée qui suit et des exemples de réalisation non limitatifs de l'invention, en référence aux figures annexées qui représentent :

Figure 1 : schéma de principe du procédé de fabrication d'acroléine selon l'invention, sur lequel l'étape de séparation de sous-produits lourds par différence de point d'ébullition est représentée en pointillés.
Figure 2 : schéma de principe du procédé de fabrication d'acide acrylique selon l'invention, sur lequel l'étape de séparation de sous-produits lourds par différence de point d'ébullition est représentée en pointillés.
Figure 3 : schéma détaillé des étapes d) et e) du procédé de fabrication d'acide acrylique selon l'invention.
Figure 4 : schéma détaillé d'un mode de réalisation préféré du procédé de fabrication d'acide acrylique selon l'invention.
Figure 5 : schéma détaillé d'un mode de réalisation préféré du procédé de fabrication d'acroléine selon l'invention.

Description détaillée de l'invention

Fabrication de l'acroléine

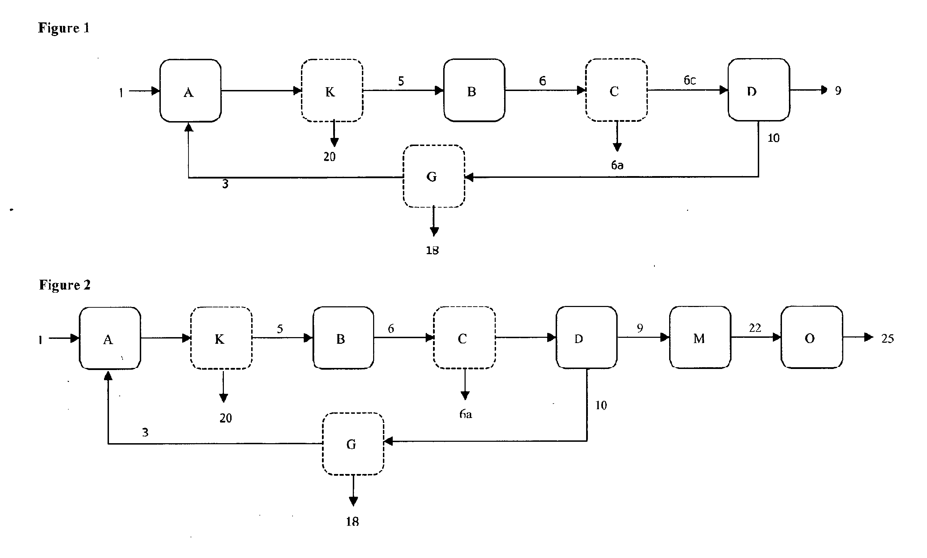

La Figure 1 est une représentation simplifiée du procédé de fabrication d'acroléine selon l'invention, qui indique, en pointillés, les différentes variantes possibles pour l'étape de séparation des sous-produits lourds insérées dans le schéma de principe d'un procédé de l'art antérieur.
En référence à la Figure 1, on utilise généralement pour alimenter le réacteur (B) de l'étape a) de déshydratation de glycérol, un flux (5) contenant le glycérol et de l'eau, avec un ratio massique eau/glycérol pouvant varier dans de larges mesures, par exemple entre 0,04 / 1 et 9 / 1, et de préférence entre 0,7 / 1 et 5 / 1. Le flux (5) peut également contenir de l'oxygène, de l'azote, de l'argon, du CO et du $CO_2$.

Ce flux (5) est avantageusement obtenu lors d'une étape de mélange (A) d'un flux (1) riche en glycérol et d'une phase (3) riche en eau recyclée pouvant contenir de l'azote, de l'oxygène, du glycérol ou des impuretés organiques du procédé. Le flux (1) peut être par exemple du glycérol brut commercial (glycérine), c'est-à-dire contenant typiquement 80-90% de glycérol, 1 à 10% de sels, 1 à 4% de matières organiques non glycérineuses dont le méthanol, et 3 à 15% d'eau. Avantageusement, on utilise du glycérol dessalé, qui peut être obtenu à partir de glycérol brut par tout moyen connu de l'homme de l'art, comme une distillation sous pression réduite ou un flash sous pression réduite ou une séparation utilisant des résines échangeuses d'ion tel que décrit par exemple dans la demande EP1978009. On peut aussi partir de glycérine sans sel obtenue par des procédés de transestérification d'huiles catalysés par des catalyseurs hétérogènes. On peut aussi utiliser de la glycérine raffinée d'une pureté supérieure à 98%, 99% ou 99,5%. On peut aussi utiliser une solution aqueuse contenant de 20% à 99%, de préférence de 30% à 80% en poids de glycérol.

La réaction de déshydratation, étape a), qui est favorisée par un niveau de température élevée, est effectuée en général en phase gaz dans le réacteur (B) en présence d'un catalyseur à une température allant de 150°C à 500°C, de préférence comprise entre 250°C et 350°C et une pression comprise entre $10^5$ et $5.10^5$ Pa (1 et 5 bars). Elle peut également être conduite en phase liquide, dans ce cas la température est comprise entre 150°C et 350°C sous une pression allant de $5.10^5$ à $100.10^5$ Pa. De préférence, on effectue cette première étape en phase gaz.

On peut aussi l'effectuer en présence d'oxygène ou d'un gaz contenant de l'oxygène comme décrit dans les demandes WO 06/087083 et WO 06/114506. Dans ce cas, la quantité d'oxygène est choisie de façon à être en dehors du domaine d'inflammabilité en tout point de l'installation. Le rapport molaire entre l'oxygène moléculaire et le glycérol est généralement de l'ordre de 0,1 à 1,5, de préférence de 0,3 à 1,0.

La réaction de déshydratation peut aussi être mise en oeuvre dans un milieu réactionnel comportant une phase gazeuse contenant de 1 et 3 000 ppm d'un composé acide au sens de la classification de Pearson choisi par exemple parmi $SO_3$, $SO_2$, $NO_2$, la réaction de déshydratation étant conduite soit en phase gaz soit en phase liquide.

La réaction de déshydratation du glycérol est généralement effectuée sur des catalyseurs solides acides. Les catalyseurs qui conviennent sont des matériaux homogènes ou multiphases, insolubles dans le milieu réactionnel qui ont une acidité de Hammett, notée $H_0$ inférieure à +2. Comme indiqué dans le brevet US 5,387,720 qui fait référence à l'article de K. Tanabe et al dans "Studies in Surface Science and Catalysis", Vol 51, 1989, chap 1 et 2, l'acidité de Hammett est déterminée par titration amine à l'aide d'indicateurs ou par adsorption d'une base en phase gazeuse.

Ces catalyseurs peuvent être choisis parmi des matériaux siliceux naturels ou de synthèse ou les zéolithes acides ; des supports minéraux, tels que des oxydes, recouverts par des acides inorganiques, mono, di, tri ou polyacides ; des oxydes ou oxydes mixtes ou encore des hétéropolyacides ou sels d'hétéropolyacides.

Ces catalyseurs pourront en particulier être constitués par un sel d'hétéropolyacide dans lequel des protons dudit hétéropolyacide sont échangés avec au moins un cation choisi parmi les éléments appartenant aux Groupes I à XVI de la Classification Périodique des Eléments, ces sels d'hétéropolyacide contenant au moins un élément choisi parmi le groupe comprenant W, Mo et V.

Parmi les oxydes mixtes, on peut citer particulièrement ceux à base de fer et de phosphore et ceux à base de césium, phosphore et tungstène.

Les catalyseurs sont notamment choisis parmi les zéolithes, les composites Nafion® (à base d'acide sulfonique de polymères fluorés), les alumines chlorées, les acides et sels d'acides phosphotungstiques et/ou silicotungstiques, et différents solides de type oxydes métalliques tels que oxyde de tantale $Ta_2O_5$, oxyde de niobium $Nb_2O_5$, alumine $Al_2O_3$, oxyde de titane $TiO_2$, zircone $ZrO_2$, oxyde d'étain $SnO_2$, silice $SiO_2$ ou silico-aluminate $SiO_2$-$Al_2O_3$, imprégnés de fonctions acides telles que borate $BO_3$, sulfate $SO_4$, tungstate $WO_3$, phosphate $PO_4$, silicate $SiO_2$, ou molybdate $MoO_3$, ou un mélange de ces composés.

Les catalyseurs précédents peuvent comprendre en plus un promoteur tel que Au, Ag, Cu, Pt, Rh, Pd, Ru, Sm, Ce, Yt, Sc, La, Zn, Mg, Fe, Co, Ni, or montmorillonite.

Les catalyseurs préférés sont les zircones phosphatées, les zircones tungstées, les zircones silicées, les oxydes de titane ou d'étain imprégnés de tungstate ou phosphotungstate ou silicotungstate, les alumines ou silices phosphatées, les hétéropolyacides ou sels d'hétéropolyacides, les phosphates de fer et les phosphates de fer comprenant un promoteur.

On peut aussi effectuer la réaction de déshydratation du glycérol en présence d'une quantité d'hydrogène allant de 0,1 à 10% en volume par rapport au mélange réactionnel, et dans ce cas en présence d'un catalyseur choisi parmi ceux décrit dans la demande US 2008/018319.

Le réacteur (B) utilisé peut fonctionner en lit fixe, en lit mobile, en lit fluidisé ou en lit fluidisé circulant, ou dans une configuration en modules (plaques ou paniers). La durée de contact exprimée en secondes est le rapport entre le volume du lit de catalyseur et le volume des réactifs gazeux envoyés par seconde. Les conditions moyennes de température et de pression existant dans un lit peuvent varier selon la nature du catalyseur, la nature du lit catalytique et la dimension du catalyseur. En général, la durée de contact est de 0,1 à 20 secondes et de préférence de 0,3 à 15 secondes.

A l'issue de l'étape a), on obtient un flux aqueux (6), pouvant être liquide ou gazeux, contenant l'acroléine recherchée, de l'eau, du glycérol n'ayant pas réagi, et des sous-produits tels que hydroxypropanone, propanaldéhyde, acétaldéhyde, formaldéhyde, acide acrylique, acide propionique, acide acétique, acide formique, acétone, phénol, des produits d'addition de l'acroléine sur le glycérol, des produits de polycondensation du glycérol, des éthers de glycérol cycliques, ainsi que des composés légers tels que azote, oxygène, monoxyde et dioxyde de carbone. Certains de ces produits sont des composés lourds, d'autres sont des composés légers condensables. Pour d'autres, il s'agit de composés légers incondensables dans les conditions de températures et de pression habituellement mises en oeuvre.

Le flux (6) a une teneur en eau importante due à la composition du flux (5) entrant dans le réacteur (charge de glycérol) et à la réaction elle-même (déshydratation). L'étape b) dans le procédé selon l'invention consiste à séparer ce flux (6) en une phase (9) enrichie en acroléine et une phase (10) riche en eau et appauvrie en acroléine. Cette étape b), telle que la condensation partielle de l'eau décrite par exemple dans la demande de brevet WO 08/087315 au nom de la Société Déposante, ou telle que la séparation décrite dans la demande WO 2006/136336, a pour but d'éliminer la majeure partie de l'eau présente et les sous-produits lourds avant d'envoyer le flux contenant l'acroléine vers une étape de purification dans un procédé de fabrication d'acroléine ou à l'étape d'oxydation de l'acroléine en acide acrylique dans un procédé de fabrication d'acide acrylique à partir de glycérol en deux étapes. Cette séparation partielle de l'eau permet ainsi d'éviter une dégradation du catalyseur de 2ème étage d'oxydation de l'acroléine en acide acrylique, et d'éviter lors des étapes ultérieures l'élimination de grandes quantités d'eau, qui risque d'être coûteuse et d'entraîner des pertes en acide acrylique. En outre, elle permet d'éliminer une partie des impuretés « lourdes » formées lors de la déshydratation du glycérol.

Cette étape b) est réalisée sur une unité de séparation (D) dans des conditions telles que 20% à 95% massiques de l'eau présente dans le flux issu de l'étape a) sont éliminés dans la phase appauvrie en acroléine. Dans le cas où l'étape a) a été réalisée en phase gaz, l'unité de séparation (D) est une installation de condensation qui peut comprendre une colonne d'absorption couplée ou non à un évaporateur, un ou des échangeurs de chaleur, un ou des condenseurs, un déflegmateur, ainsi que tout appareillage bien connu de l'homme de l'art permettant de réaliser une condensation partielle d'un flux aqueux. Elle est conduite dans des conditions telles que 20% à 95%, de préférence 40% à 90% de l'eau présente dans le flux (6) est éliminée dans le flux (10) liquide. Le flux gazeux (9) contient généralement plus de 80% et de préférence plus de 90% de l'acroléine initialement contenue dans le flux (6). Ce résultat est obtenu en abaissant la température à une température de 60 à 120°C.

Dans le cas où l'étape a) a été réalisée en phase liquide sous pression, l'étape b) peut être réalisée par une détente à une pression de 1 à 4 bars éventuellement couplée à un échangeur de chaleur et une installation de séparation gaz liquide qui peut être un ballon de flash, une colonne à distiller ou tout autre dispositif connu de l'homme de l'art. On récupère un flux liquide (10) qui contient 20% à 95%, de préférence 40% à 90% de l'eau présente dans le flux (6) et un flux gazeux (9) qui contient plus de 80% et de préférence plus de 90% de l'acroléine initialement contenue dans le flux (6).

La phase condensée (10) ainsi générée contient généralement de 90% à 99% d'eau, le reste représentant de l'acroléine et des impuretés telles que acide acrylique, glycérol, acide acétique, hydroxypropanone, acide propionique, et autres composés organiques plus lourds que l'eau.

Un des objets du procédé de l'invention est d'obtenir une phase aqueuse riche en eau et appauvrie en acroléine (10) qui soit recyclable au moins en partie à l'étape de réaction sous forme de flux (3), exempte d'impuretés lourdes néfastes pour le catalyseur de déshydratation.

Pour cela, il est mis en oeuvre une étape d'élimination des composés organiques plus lourds que l'eau, soit au niveau du flux (5) entrant dans le réacteur de déshydratation (B), soit sur le flux aqueux (6) sortant du réacteur de déshydratation (B), soit encore sur la phase aqueuse (10) riche en eau et appauvrie en acroléine sortant de l'unité de séparation (D), ces variantes pouvant être utilisées seules ou en combinaison. On obtient une boucle fermée d'eau évitant ainsi une trop grande consommation d'eau dans le procédé.

L'élimination des composés organiques plus lourds que l'eau est réalisée par différence des points d'ébullition de ces composés, soit par évaporation lorsque le flux à séparer est initialement liquide, soit par condensation lorsque le flux à séparer est initialement gazeux, soit par une combinaison d'évaporation et de condensation lorsqu'on utilise plusieurs étages de séparation.

Selon un premier mode de réalisation de l'invention, l'étape d'élimination des composés lourds est réalisée par évaporation du mélange de glycérol (1) et de la phase riche en eau recyclée (3), dans un appareil (K), qui génère une phase riche en glycérol et en eau et appauvrie en composés lourds (flux (5)), qui est envoyée dans le réacteur de déshydratation (B), et une phase (20) liquide ou solide riche en composés organiques plus lourds que l'eau qui est éliminée.

Selon un second mode de réalisation de l'invention, l'étape d'élimination des composés lourds est réalisée par un traitement du flux (6) sortant du réacteur de déshydratation (B), dans un appareil (C), qui génère une phase gaz (6c) appauvrie en composés organiques plus lourds que l'eau et une phase (6a) liquide ou solide enrichie en composés organiques plus lourds que l'eau qui est éliminée. Dans le cas d'une réaction de déshydratation en phase gaz, l'appareil (C) réalisera un refroidissement du flux (6) de façon à générer une phase condensée liquide (6a) et une phase (6c) gaz.

Dans le cas d'une réaction de déshydratation en phase liquide, l'appareil (C) permettra de vaporiser partiellement le flux (6) par détente ou par chauffage en un flux gaz (6c) et un flux liquide ou solide (6a).

Selon un troisième mode de réalisation de l'invention, l'étape d'élimination des composés lourds est réalisée par évaporation du flux (10) sortant de l'unité de séparation (D), dans un appareil (G), qui génère une phase gaz (3) riche en eau qui est recyclée, et une phase (18) liquide ou solide enrichie en composés organiques plus lourds que l'eau qui est éliminée.

Comme appareils (K) et (G), on peut utiliser des évaporateurs, ou des colonnes à distiller. Tout type d'évaporateur connu de l'homme de l'art est envisageable, tels des évaporateurs à double enveloppe, à serpentin, ou à surface d'échange tubulaire horizontale, verticale ou inclinée, à convection naturelle ou circulation forcée, à film agité ou raclé, ou des évaporateurs à plaques. On peut également utiliser des colonnes à distiller à plateaux ou des colonnes comportant des garnissages structurés ou vrac. Avantageusement, ces colonnes comportent une zone de vaporisation ou de rebouillage sur laquelle des calories peuvent être apportées par tout moyen connu par l'homme du métier tels des échangeurs à thermosiphon ou à circulation forcée fonctionnant à la vapeur ou des réchauffeurs électriques. Ces colonnes peuvent comporter un ou des échangeurs de refroidissement destinés à faire du reflux et augmenter les capacités de séparation des produits plus lourds que l'eau à séparer.

Les appareils (K) pourront avoir, outre leur rôle de séparation de produits lourds, le rôle de vaporisation du glycérol. On cherchera avantageusement à réduire le temps de séjour et la température de paroi des échangeurs avec lesquels le mélange glycérol / eau est en contact. Une circulation forcée pourra être avantageuse. La température de fonctionnement des appareils (K) sera avantageusement autour du point d'ébullition du glycérol dans les conditions de pression et de composition de l'installation, soit typiquement entre 180°C et 320°C et de préférence entre 200°C et 270°C. Avantageusement, les appareils (K) peuvent comporter une section d'épuisement des lourds en glycérol, de façon à éviter trop de perte en glycérol.

Les appareils de type (G) fonctionneront de préférence au voisinage du point d'ébullition de l'eau dans les conditions de pression et de composition du flux entrant, soit typiquement de 100°C à 180°C et de préférence de 110°C à 150°C.

Comme appareils (C), on peut utiliser un contacteur constitué d'une colonne vide ou une colonne garnie possédant une boucle de recirculation externe prélevant la phase liquide en pied de ladite colonne, la refroidissant dans un échangeur de refroidissement et la renvoyant en tête de ladite colonne. Dans le cas d'un fût vide, la phase liquide refroidie sera pulvérisée en tête de la colonne de façon à faciliter les échanges thermiques. La température de ladite phase liquide sera typiquement de 120°C à 180°C et de préférence de 140°C à 170°C. Avantageusement, le débit de recirculation sera réglé de façon à obtenir une différence de température faible entre l'entrée et la sortie de l'échangeur de refroidissement, typiquement inférieure à 30°C et de préférence inférieure à 10°C.

L'appareil (C) peut aussi être constitué d'un échangeur à couche mine, comme par exemple un échangeur à film raclé.

L'appareil (C) peut aussi être constitué d'un échangeur multitubulaire comprenant une séparation gaz-liquide en sortie.

Alternativement, un liquide peut être ajouté à la phase gaz dans l'une des solutions techniques présentées précédemment de façon à augmenter le contact avec les surfaces d'échange. On choisira un liquide à point d'ébullition supérieur au point de fonctionnement de l'appareil (C), tel par exemple le glycérol.

Avantageusement, on placera un échangeur sur la phase gaz entre la sortie du réacteur (B) et l'entrée de (C) qui permet de refroidir sans condenser la phase gaz et de récupérer des calories. En sortie de cet échangeur, la température est typiquement de 150°C à 250°C et de préférence 180°C à 230°C.

Dans tous les modes de réalisation de l'invention, on obtient un flux (3) qui peut être avantageusement recyclé totalement ou en partie à l'étape a) de déshydratation du glycérol, sans risque d'accumulation d'impuretés sur le catalyseur de déshydratation ; ce flux (3) permet notamment d'ajuster la teneur en eau du flux (5) contenant le glycérol qui va alimenter le réacteur de déshydratation (B). Avantageusement le flux (3) peut être surchauffé afin de permettre la vaporisation du flux de glycérol dans l'étape de mélange (A) avant d'envoyer le flux gazeux réactif dans le réacteur de déshydratation (B). On évite ainsi d'avoir à mettre en contact le glycérol avec une paroi très chaude, nécessaire pour le vaporiser, puisque toutes les calories sont apportées par le flux (3) surchauffé.

Les phases liquides ou solides enrichies en composés organiques plus lourds que l'eau sont, soit éliminées, soit envoyées dans un oxydeur thermique pour éliminer les composés à l'état de $CO_2$ et $H_2O$.

Dans la mesure où la réaction de déshydratation du glycérol en acroléine produit 2 molécules d'eau par molécule d'acroléine produite, une purge sur le flux (3) peut être prévue de façon à éviter l'accumulation d'eau dans la boucle. Cette purge pourra être éliminée, soit directement, soit via un dispositif de traitement d'eau tel une station biologique ou un traitement oxydant. Cette purge sera plus ou moins importante selon la quantité d'eau qui est emportée dans la phase enrichie en acroléine (9). La purge d'eau peut avantageusement être effectuée au niveau de l'appareil (G), en laissant partir une quantité adaptée d'eau avec les sous-produits organiques plus lourds que l'eau dans le flux (18).

Selon un mode de réalisation de l'invention, le flux (6a) provenant de l'appareil (C) peut être réinjecté dans le flux (10) en amont de l'appareil (G) ou à l'étape de mélange (A) en amont de l'unité (K), de façon à recycler le glycérol non converti dans le réacteur de déshydratation (B). Dans ces conditions, les lourds séparés du flux (6) par l'unité (C) seront effectivement éliminés de la boucle d'eau au niveau des unités (G) ou (K).

Selon un mode de réalisation du procédé selon l'invention (non représenté sur la figure 1), la phase (9) enrichie en acroléine, qui est débarrassée des sous-produits lourds et d'une grande partie de l'eau, provenant de l'étape b) de séparation du flux issu de l'étape de déshydratation a), est soumise à un traitement de purification comprenant des étapes d'absorption/distillation, telles que celles décrites par exemple pour le flux d'acroléine produit par oxydation du propylène dans le document Techniques de l'Ingénieur, Traité des Procédés, J 6 100 1-4.

La purification du flux (9) contenant l'acroléine, après refroidissement par un ou plusieurs échangeurs de chaleur, comprend en général une absorption dans de l'eau ou un flux aqueux recyclé pour laisser partir en tête les incondensables et récupérer en pied une solution aqueuse d'acroléine diluée.

Cette absorption peut être réalisée dans une colonne à garnissage ou à plateaux, de préférence à contre-courant. Avantageusement, on élimine en tête de la colonne les composés légers incondensables, tels que azote, oxygène, monoxyde et dioxyde de carbone.

La solution aqueuse d'acroléine est ensuite séparée par distillation. Pour cela, on peut utiliser un enchaînement de colonnes à distiller, comme décrit par exemple dans le brevet US 3,433,840 ou une seule colonne comme décrit par exemple dans les documents EP1300384 ou EP1474374. Cette distillation permet de récupérer, d'une part un flux constitué majoritairement d'eau dont la majeure partie est généralement recyclée à l'étape d'absorption, et d'autre part un flux gazeux ou liquide contenant une teneur massique en acroléine supérieure à 80% et de préférence > 94% et une teneur massique en eau inférieure à 15% par rapport à l'acroléine et de préférence < 5%.

La purification du flux (9) contenant l'acroléine peut aussi être réalisée simplement par distillation sans absorption préalable dans l'eau. Cette alternative est avantageusement mise en oeuvre lorsque le flux (9) contient peu de gaz incondensables.

Le flux d'acroléine, liquide ou gazeux, obtenu à l'issu des étapes de purification du flux (9), peut alors être utilisé pour préparer du méthylmercaptopropionaldéhyde (MMP) par réaction avec du méthyl mercaptan en présence d'un catalyseur. La réaction du MMP, éventuellement purifié, avec de l'acide cyanhydrique ou du cyanure de sodium effectuée selon les synthèses de Bücherer ou de Strecker bien connues de l'homme du métier, conduit alors, soit à la méthionine, soit à l'hydroxyanalogue de la méthionine, après transformation du produit de réaction, comme décrit dans le document Techniques de l'Ingénieur, Traité Génie des procédés, J 6 410-1 à 9.

Fabrication de l'acide acrylique

En référence à la figure 2, pour la mise en oeuvre du procédé selon l'invention, la phase (9) enrichie en acroléine et débarrassée des sous-produits lourds et d'une grande partie de l'eau, provenant de l'étape b) de séparation du flux issu de l'étape de déshydratation a), est soumise à une réaction d'oxydation catalytique d) dans un réacteur (M) pour obtenir un flux (22) contenant l'acide acrylique recherché. Ce flux est ensuite soumis dans une étape e) à un ou plusieurs traitements de purification (O) permettant de récupérer de l'acide acrylique purifié (25).

La réaction d'oxydation de l'acroléine en acide acrylique s'effectue en présence d'oxygène moléculaire ou d'un mélange contenant de l'oxygène moléculaire, à une température allant de 200°C à 350°C, de préférence de 250°C à 320°C, et sous une pression allant de 1 à 5 bars en présence d'un catalyseur d'oxydation. Comme catalyseur d'oxydation, on utilise tous types de catalyseurs bien connus de l'homme de l'art pour cette réaction. Généralement sont utilisés des solides contenant au moins un élément choisi dans la liste Mo, V, W, Re, Cr, Mn, Fe, Co, Ni, Cu, Zn, Sn, Te, Sb, Bi, Pt, Pd, Ru, Rh, présent sous la forme métallique ou sous forme d'oxyde, de sulfate ou de phosphate. En particulier, sont utilisées les formulations contenant Mo et/ou V et/ou W et/ou Cu et/ou Sb et/ou Fe comme constituants principaux.

Le réacteur d'oxydation (M) peut fonctionner en lit fixe, en lit fluidisé ou en lit fluidisé circulant. Il est possible aussi d'utiliser un échangeur à plaques avec un agencement modulaire du catalyseur tel que décrit dans les documents EP 995491, EP 1147807 ou US 2005/0020851.

Le mélange gazeux (22) issu de la réaction d'oxydation est constitué, en dehors de l'acide acrylique de différents composés tels que :

- des composés légers incondensables dans les conditions de températures et de pression habituellement mises en oeuvre : $N_2$, $O_2$ non converti, CO et $CO_2$ formés en faible quantité par oxydation ultime ou tournant en rond, par recyclage, dans le procédé,
- de composés légers condensables : en particulier l'eau résiduelle de l'étape précédente, générée par la réaction de déshydratation ou présente comme diluant, l'acroléine non convertie, des aldéhydes légers, comme le formaldéhyde et l'acétaldéhyde, l'acide formique et l'acide acétique et l'acide propionique.
- des composés lourds résiduels de l'étape précédente: furfuraldéhyde, benzaldéhyde, acide et anhydride maléique, acide benzoïque, phénol, protoanémonine.

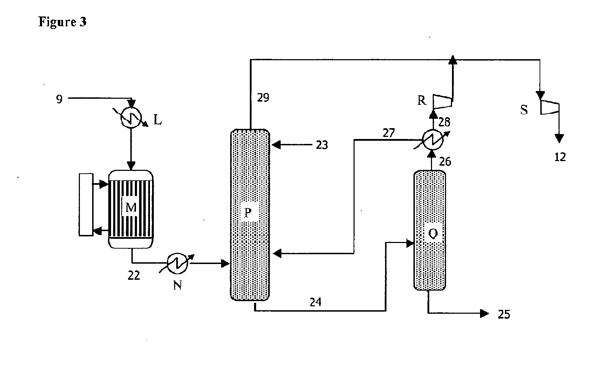

Pour obtenir de l'acide acrylique répondant à une certaine qualité technique, il est nécessaire de soumettre ce mélange (22) à un train de récupération/purification, représenté en partie par exemple sur la figure 3 :

La première étape de cette phase de purification consiste en une extraction de l'acide acrylique par absorption à contre-courant. Pour cela, on introduit le mélange gazeux (22), éventuellement après refroidissement dans un échangeur (N) en pied d'une colonne d'absorption (P) où il rencontre à contre-courant un solvant (23) introduit en tête de colonne, généralement de l'eau. Les composés légers incondensables dans les conditions de température et de pression habituellement mises en oeuvre (respectivement plus de 50°C et moins de $2.10^5$ Pa) sont éliminés en tête de cette colonne d'absorption dans un flux (29). Le solvant (23) mis en oeuvre dans cette colonne est l'eau. L'eau pourrait être remplacée par un solvant hydrophobe à haut point d'ébullition, comme il est décrit par exemple dans les brevets de BASF FR 2.146.386 ou US 5.426.221, ainsi que dans le brevet FR 96.14397. L'eau utilisée comme solvant absorbant peut être apportée par une source extérieure au procédé, mais peut être constituée pour partie ou totalement par de l'eau issue du recyclage d'une phase aqueuse dans le procédé, par exemple l'eau séparée dans l'unité de séparation (D), ou l'eau récupérée à partir du flux de tête d'une colonne de séchage azéotropique éventuellement présente dans le train de purification. Selon une variante, il n'est pas ajouté d'eau extérieure au procédé dans la colonne d'absorption. Les conditions opérationnelles de cette étape d'absorption sont les suivantes :

Le mélange réactionnel gazeux est introduit en pied de colonne à une température comprise entre 130°C et 250°C. L'eau est introduite en tête de colonne à une température comprise entre 10°C et 60°C. Les quantités respectives d'eau et de mélange réactionnel gazeux sont telles que le ratio massique eau / acide acrylique est compris entre 1/1 et 1/4. L'opération est conduite à la pression atmosphérique.

La colonne d'absorption (P) peut être couplée avec une colonne de distillation des composés très légers, essentiellement l'acroléine non convertie à l'issue de la réaction, présente en faible concentration dans la solution aqueuse d'acide acrylique récupérée en pied de colonne d'absorption. Cette colonne de distillation (Q), fonctionnant sous une pression de $6.10^3$ à $7.10^4$ Pa, est alimentée en tête par le flux (24) de pied de colonne d'absorption précédente, et permet d'éliminer en tête un flux (26) d'acide acrylique enrichi en acroléine, qui est recyclé au moins partiellement, via un condenseur, sous forme d'un flux liquide (27) en partie inférieure de la colonne d'absorption, pour une élimination finale en tête de cette même colonne, le flux gazeux restant (28) étant recomprimé dans un compresseur (S) et envoyé éventuellement dans l'oxydeur présent dans le procédé ou bien est recyclée dans le réacteur (B) de déshydratation ou à l'étape (D) de séparation. On obtient ainsi à l'issue de ces étapes de purification un mélange (25) d'acide acrylique dans le solvant, de préférence dans l'eau (ratio massique 1/4 à 4/1) débarrassé de l'essentiel de l'acroléine non convertie, que l'on nomme "acide acrylique brut".

Selon la qualité recherchée pour l'acide acrylique, ce mélange sera soumis à des traitements complémentaires, décrits dans de nombreux brevets, notamment à une étape de déshydratation qui est réalisée en présence d'un solvant de l'acide acrylique non miscible à l'eau. Cette étape de déshydratation peut être réalisée par distillation azéotropique du mélange solvant, eau, acide acrylique qui permet de sortir en tête de distillation l'azéotrope solvant / eau. L'acide acrylique récupéré en pied subit ensuite une distillation des composés légers (étêtage) et séparation des composés lourds (équeutage). On obtient alors une qualité d'acide acrylique dénommée « technique », qui peut ensuite subir une purification ultérieure, par exemple par cristallisation fractionnée pour donner un grade glacial.

Des exemples de purification de l'acide acrylique ont été décrits dans les demandes de brevet WO 10/31949, FR 09.55111, FR 09.55112 ou FR 10.51961.

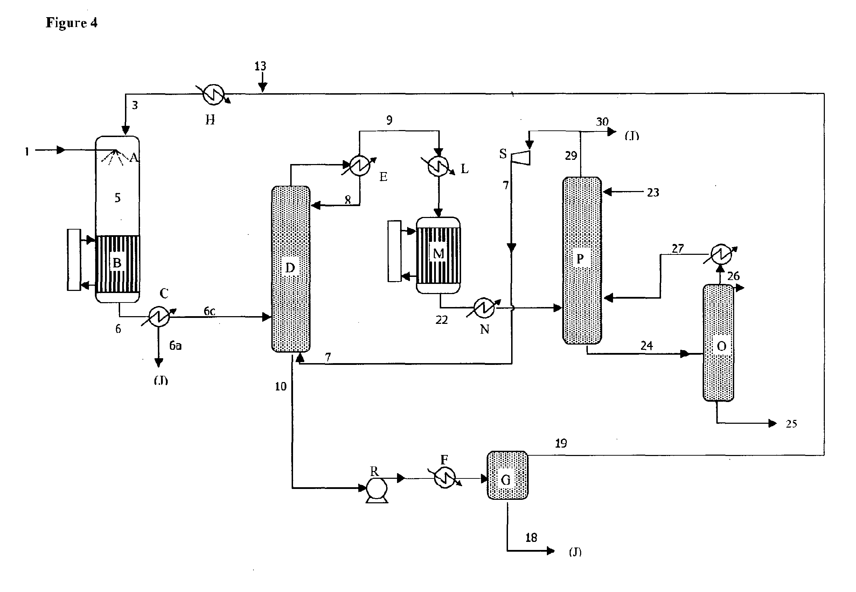

Selon un mode de réalisation préféré du procédé de fabrication d'acide acrylique selon l'invention, la réaction de déshydratation du glycérol est réalisée en phase gaz, et on applique une étape de séparation de sous-produits lourds par différence de points d'ébullition au flux aqueux sortant de l'étape a) et à la phase aqueuse appauvrie en acroléine de l'étape b). Ce mode de réalisation est illustré par exemple par la figure 4 :

Le flux gazeux (5) alimentant le réacteur de déshydratation (B) est obtenu dans la chambre de mélange (A) dans laquelle s'effectue la vaporisation du glycérol à l'aide de gaz chauds issus du recyclage (3) de la phase aqueuse (19) issue de l'unité (G), après élimination des impuretés lourdes (18) présentes dans le flux (10) appauvri en acroléine sortant en pied de l'unité de séparation (D). Le flux (1) de glycérol sous forme liquide, éventuellement préchauffé à une température de l'ordre de 100°C à 250°C peut être injecté dans cette chambre dans une veine gazeuse à co-courant ou à contre-courant via des buses de pulvérisation ou d'atomisation qui permettent de former de fines gouttelettes au contact du flux (3) recyclé comprenant essentiellement de l'eau, de l'oxygène et du $CO_2$ et éventuellement de l'azote et du CO. Les buses de pulvérisation permettent de disperser le flux de glycérol liquide sous forme de fines gouttelettes en jouant sur des effets mécaniques (taille et forme d'orifice de la buse, débit, pression). Ces systèmes permettent de former des gouttelettes de taille inférieure au mm, et de préférence inférieure

à 300 μm. Plus la taille des gouttelettes est fine, plus l'évaporation du flux (1) est rapide. Dans le schéma de la figure 4 l'énergie nécessaire à préchauffer le flux (5) entrant dans le réacteur est apportée par le flux (3) surchauffé. Alternativement un échangeur de chaleur pourrait être placé entre l'unité de mélange (A) et le réacteur (B).

Un apport d'oxygène, d'air ou d'un gaz contenant de l'oxygène favorisant la réaction de déshydratation est effectué via un flux (13) ou directement dans la chambre de mélange (A).

Le flux réactionnel gazeux (6) à la sortie du réacteur est refroidi dans un échangeur de chaleur (C) à une température comprise entre 120°C et 180°C, de préférence entre 140°C et 170°C, pour former un flux liquide (6a) de produits lourds et un flux gazeux (6c) qui est envoyé dans une colonne de condensation (D), équipée d'un condenseur (E) qui permet de séparer, d'une part une phase liquide (8) contenant majoritairement de l'eau et de l'acroléine qui est recyclée dans la colonne (D), et d'autre part le flux gazeux (9) contenant l'acroléine produite. Généralement ce flux (9) contient de l'eau dans un ratio massique acroléine / eau allant de 1/0,02 à 1/3 et de préférence 1/0,5 à 1/2, mais aussi les sous-produits légers, tels que acétaldéhyde, propanaldéhyde, acétone et éventuellement $O_2$ et des gaz inertes azote, CO et $CO_2$. De façon à épuiser en acroléine le flux liquide (10) sortant en pied de la colonne (D), celle-ci peut être alimentée en pied par un flux gazeux (7) issu de la tête de la colonne d'absorption (P) de l'acide acrylique dans l'eau. Alternativement, l'épuisement de l'acroléine peut être réalisé par rebouillage en pied de la colonne (D) ou par l'adjonction d'une colonne de stripping alimentée en tête par le flux liquide (10) et en pied par un flux gazeux comme décrit précédemment.

Le flux liquide (10) sortant en pied de la colonne d'absorption (D) est pompé dans l'échangeur (F) pour le porter à une température allant de 100°C à 180°C, de préférence de 110°C à 150°C, sous une pression de 1 à 5 bars, puis envoyé dans l'unité (G) pour subir une vaporisation. Le résidu liquide de vaporisation (18), concentré en organiques plus lourds que l'eau est, soit injecté dans un oxydeur (J), soit éliminé du procédé et traité par tout moyen connu de l'homme de l'art. La phase gaz (19), qui contient principalement de l'eau avec un peu de sous-produits légers est mélangée avec un flux d'air (13) et portée grâce à l'échangeur (H) à une température pouvant aller de 350°C à 550°C, et de préférence de 400°C à 500°C.

Le flux gazeux (9) contenant l'acroléine produite est réchauffé à une température pouvant aller de 160°C à 300°C par un échangeur (L), puis injecté dans un deuxième réacteur multitubulaire à lit fixe (M) comportant un catalyseur d'oxydation d'acroléine et un bain de sels fondus pour évacuer la chaleur dégagée par la réaction.

En sortie de ce réacteur, le flux gazeux (22) est soumis à un train de récupération/purification, tel que décrit précédemment à l'aide de la colonne d'absorption (P) et la colonne de distillation (Q). La phase gaz (29) sortant en tête de la colonne (P) est en partie recomprimée par le compresseur (S) pour former le flux (7) qui est renvoyé dans la colonne (D), l'autre partie (30) pouvant être envoyée dans un oxydeur thermique (J).

Pour une optimisation énergétique du procédé, on utilise les fumées de l'oxydeur thermique (J) comme fluide chaud pour les échangeurs thermiques, et en particulier pour l'échangeur (H).

Selon un autre mode de réalisation préféré du procédé de fabrication d'acide acrylique selon l'invention, la réaction de déshydratation du glycérol est réalisée en phase gaz, et on applique une étape de séparation de sous-produits lourds par différence de points d'ébullition au flux entrant à l'étape de déshydratation a), et au flux aqueux sortant de l'étape a). Ce mode de réalisation est illustré dans l'exemple 2 de la partie expérimentale.

Optimisation énergétique du procédé selon l'invention

Dans le procédé selon l'invention sont présents des flux gazeux qui nécessitent d'être refroidis et condensés, des flux liquides qui nécessitent d'être vaporisés. L'utilisation de systèmes à compression, tels des pompes à chaleur ou des compressions sur certains flux du procédé, permet de minimiser les calories perdues en permettant de transférer de la chaleur du milieu le plus froid vers le milieu le plus chaud. Une pompe à chaleur est un dispositif thermodynamique dont le fonctionnement est basé sur le principe de cycle à compression de fluides frigorigènes. Lorsque le fluide est comprimé et passe de l'état gazeux à l'état liquide, il se produit un phénomène exothermique (condensation) qui produit de la chaleur. A l'inverse, si on détend le fluide en le faisant passer de l'état liquide à l'état gazeux, il se produit un phénomène endothermique (évaporation) qui permet d'absorber de la chaleur et de refroidir. Tout repose sur le changement d'état utilisé en circuit fermé.

Dans le procédé de l'invention, on utilise avantageusement une pompe à chaleur pour récupérer l'énergie de condensation de l'eau du flux réactionnel gazeux (6c) à la sortie du réacteur de déshydratation et pour vaporiser la phase aqueuse appauvrie en acroléine séparée de la phase enrichie en acroléine lors de l'étape b). La pompe à chaleur pourra fonctionner avec de l'eau ou avec tout fluide frigorigène adapté connu de l'homme de l'art, tel que par exemple le 1,1,1,3,3-pentafluoropropane ou le 1,1,1,3,3-pentafluoropentane, ou une composition comprenant au moins une hydrochlorooléfine tel que le 1-chloro,3,3,3-trifluoropropène ou le 2-chloro,3,3,3-trifluoropropène, ou une composition comprenant en poids de 1 à 50% de méthyltétrahydrofuranne et de 5 à 99% de nonafluorobutyl alkyl éther de formule $C_4F_9OR$, R comportant de 1 à 4 atomes de carbone, telle que décrite dans la demande de brevet FR 2 928 648 .

Dans le cas où la réaction de déshydratation est réalisée en phase gaz, le flux (6) sort du réacteur sous forme d'un mélange gazeux à une température allant de 150°C à 550°C et de préférence entre 250°C et 400°C. Ce flux est refroidi grâce à un premier échangeur de chaleur (non représenté) pour l'amener à une température allant de 150°C à 200°C. Généralement, cet échangeur permet de récupérer de l'énergie en produisant de la vapeur basse pression. Un deuxième échangeur permet de refroidir ce flux à une température allant de 70°C à 120°C et de préférence de 90°C à 110°C, soit à l'entrée (échangeur (C)) soit en tête de la colonne d'absorption (D) (échangeur (E)), d'où il sort en pied la phase appauvrie en acroléine (10), liquide, séparée de la phase gazeuse enrichie en acroléine (9). Ce flux liquide (10) est vaporisé à une pression supérieure de 0,1 à 3 bars à la pression d'entrée du réacteur de déshydratation grâce à l'unité (G) à une température allant de 100°C à 180°C et de préférence de 110°C à 150°C.

La pompe de chaleur fonctionnant avec un fluide frigorigène pouvant être de l'eau ou tout autre fluide frigorigène est installée sur le deuxième échangeur et les échangeurs de chaleur de l'unité (G) précités. Un flux liquide est vaporisé dans le premier échangeur, puis comprimé dans un compresseur à une pression comprise entre 2 et 30 bars et de préférence 2 à 8 bars et une température de 110°C à 200°C. Le flux obtenu est condensé dans le second échangeur de chaleur puis détendu et refroidi pour redonner le flux liquide, formant ainsi une boucle entre les deux échangeurs.

Dans le cas où la réaction de déshydratation est réalisée en phase gaz, des systèmes de compression permettant une récupération d'énergie peuvent également être utilisés entre l'étape a) de réaction de déshydratation et l'étape b) de séparation, de façon à pouvoir récupérer l'énergie de condensation de l'eau contenue dans le flux réactionnel gazeux lors de l'étape b) de séparation. Dans une configuration exemplifiée par la figure 4, le flux gazeux (6) sortant du réacteur de déshydratation est refroidi par un échangeur de chaleur (C) puis recomprimé par un compresseur (non représenté sur la figure 4) avant d'être injecté dans la colonne de condensation (D). La colonne (D) et l'échangeur (E) fonctionnent à une pression supérieure d'au moins 1 bar et de préférence au moins 2 bars à celle du réacteur (B). Le flux liquide (10) récupéré en pied est ensuite vaporisé via l'échangeur F et l'unité (G) qui fonctionnent à une pression inférieure à celle de la colonne (D) et (E) d'au moins 0,5 bar et de préférence d'au moins 1,5 bar et une pression supérieure à celle du réacteur (B). La compression réalisée entre l'étape a) de déshydratation du glycérol et l'étape b) peut également être réalisée par une succession d'échangeurs de refroidissement et de compresseurs en série.

Dans ces conditions, la condensation en tête de la colonne (D) est à un niveau thermique suffisant pour que l'énergie de condensation puisse être récupérée. Cette condensation en tête de la colonne (D) peut être couplée à la vaporisation en sortie de la pompe (R), c'est-à-dire que le flux gazeux sortant en tête de la colonne peut être refroidi directement par le flux liquide sortant de la pompe (R), qui sera lui-même réchauffé. Autrement dit, les échangeurs (C), (E) et (F) et ceux des unités (G) peuvent être les mêmes échangeurs. Dans une autre configuration, la condensation en tête de la colonne (D) réalisée par l'échangeur (E) peut servir à produire de la vapeur qui pourra être utilisée à d'autres endroits dans le procédé de production de l'acroléine ou l'acide acrylique ou à l'extérieur du procédé.
Avantageusement, le flux gazeux (6) sortant du réacteur de déshydratation peut être refroidi par l'échangeur de chaleur (C) jusqu'à une température basse, typiquement de 110°C à 160°C, de façon à produire de la vapeur dans l'échangeur (C), vapeur qui pourra être utilisée à d'autres endroits dans le procédé de production de l'acroléine ou l'acide acrylique ou à l'extérieur du procédé.

Le(s) pompe(s) à chaleur mise(s) en oeuvre dans le procédé selon l'invention peu(ven)t également être utilisée(s) pour produire de la vapeur à un niveau thermique suffisant pour qu'elle(s) ai(en)t un usage dans ou à l'extérieur du procédé.

Le procédé selon l'invention contribue ainsi à la diminution de la consommation de combustibles et du rejet de $CO_2$ vers l'atmosphère.

L'acide acrylique bio-ressourcé obtenu selon le procédé de l'invention peut être utilisé pour la fabrication d'homopolymères et copolymères produits par polymérisation de l'acide acrylique et éventuellement d'autres monomères insaturés, par exemple la fabrication de polymères superabsorbants obtenus par polymérisation dudit acide partiellement neutralisé, ou la polymérisation dudit acide suivie d'une neutralisation partielle de l'acide polyacrylique obtenu.

L'acide acrylique bio-ressourcé obtenu selon le procédé de l'invention peut être utilisé aussi pour la fabrication de polymères ou de copolymères par polymérisation des dérivés dudit acide sous forme ester ou amide.

PARTIE EXPERIMENTALE

Une simulation à l'aide du logiciel ASPEN a été utilisée dans les exemples 1 et 2 pour illustrer le procédé selon l'invention. Les pourcentages sont exprimes en % massiques. Les pressions sont exprimées en bar absolu. On ne mentionnera pas les espèces dont la teneur est inférieure à 1%.

**EXEMPLE 1 (en référence à la figure 4) :** Déshydratation en phase gaz du glycérol pour produire de l'acroléine qui est oxydée en acide acrylique et séparation de produits lourds par condensation juste après l'étape de déshydratation de glycérol et séparation de produits lourds par flash sur la boucle d'eau.

Un flux liquide (1) de glycérol préchauffé à 220°C (17,4 T/h, 99,0% glycérol) est injecté via une buse de pulvérisation (A) dans un flux gazeux recyclé (3) (65,2 T/h, 478°C, 2,8 bars, 63,6% eau, 24,7% $N_2$, 7,4% $O_2$, 1,4% acide acrylique, 1,2% acide acétique). La pulvérisation du glycérol en fines gouttelettes permet sa vaporisation sur une courte distance.

Le flux gazeux résultant (5) (79,2 T/h, 320°C, 2,8 bars, 21,7% glycérol, 49,8% eau, 19,3% azote, 5,8% oxygène, 1,3% acide acrylique), est envoyé dans un réacteur multitubulaire à lit fixe (B) contenant 35 m' d'un catalyseur hétérogène acide de déshydratation et couplé à un bain de sel fondu qui permet d'évacuer la chaleur produite par les réactions. De ce réacteur sort un flux gazeux (6) à 320°C sous 1,7 bar (58,2% eau, 19,3% $N_2$, 4,6% oxygène, 10,6% acroléine, 1,0% acétaldéhyde, 1,5% acide acrylique, 1,1% acide acétique). Ce flux est refroidi à 160°C dans un échangeur de chaleur (C), qui permet de récupérer un flux liquide (6a) de produits lourds (68 kg/h) et une fraction gazeuse (6c). Le flux liquide est envoyé vers un oxydeur thermique (J). La fraction gazeuse est envoyée dans une colonne d'absorption (D). On injecte en pied de la colonne (D) un flux gazeux (7) (21,8 T/h, 138°C, 65,3% $N_2$, 22,0% eau, 3,7% $CO_2$, 3,1% CO, 2,6% $O_2$). La colonne (D) comporte en tête un condenseur partiel (E). De ce condenseur partiel (E) sort une phase liquide (8) (75°C, 48,4 T/h) qui est renvoyée dans la colonne (D) et un flux gazeux enrichi en acroléine (9) (54,6 T/h) qui sort à 75°C sous 1,7 bar et contient 54,6% de $N_2$, 15,6% d'acroléine, 14,0% d'eau, 7,7% d'$O_2$, 2,4% $CO_2$, 2,4% CO, 1,6% d'acétaldéhyde.

Le flux gazeux (9) est réchauffé à 240°C par un échangeur (L), puis injecté dans un deuxième réacteur multitubulaire à lit fixe (M) comportant un catalyseur d'oxydation et un bain de sels fondus pour évacuer la chaleur dégagée par la réaction. En sortie de ce réacteur, le flux gazeux (22) (54,6 T/h, 54,0% $N_2$, 19,0% acide acrylique, 14,3% eau, 3,0% $CO_2$, 2,6% monoxyde de carbone, 2,1% oxygène, 1,5% acide acétique) est refroidi à 160°C par l'échangeur (N), puis injecté dans la colonne d'absorption (P). En tête de cette colonne, on injecte un flux (23) de 6,0 T/h d'eau à 25°C. On récupère en pied une phase liquide (24) qui est envoyée vers une colonne (Q) fonctionnant sous vide qui permet de récupérer un flux d'acide acrylique (25) (15,4 T/h, 66,2% acide acrylique, 25,0% eau, 5,1% acide acétique, 3,1% acide formique). En tête de la colonne (Q), le flux gazeux (26) (1,6 T/h, 72°C, 0,3 bars) est envoyé dans un condenseur puis dans la colonne (P). La phase gaz (29) sortant en tête de la colonne (P) (45,2 T/h, 72°C, 1,1 bar, 65,3% $N_2$, 22,0% eau, 3,7% $CO_2$, 3,1% CO, 2,6% $O_2$) est en partie recomprimée à 1,8 bar par le compresseur (S) et forme le flux (7) décrit plus haut. L'autre partie (30) est envoyée dans un oxydeur thermique (J).

Le flux liquide (10) appauvri en acroléine sortant en pied de la colonne (D) (46,3 T/h, 86°C, 1,8 bars, 93,4% d'eau, 0,04% d'acroléine, 1,9% d'acide acétique, 2,6% d'acide acrylique, 1,0% d'hydroxypropanone, 1,1% d'autres composés organiques lourds) est pompé dans un échangeur (F) qui le porte à 132°C sous 2,8 bars, puis flashé dans le ballon de flash (G) pour donner une phase liquide (18) (4,6 T/h) et une phase gaz (19) (41,7 T/h, 94,3% eau, 2,5% d'acide acrylique, 1,5% d'acide acétique). Cette phase gaz (19) est mélangée avec un flux d'air (13) de 20,2 T/h à 166°C sous 2,8 bar puis chauffée à 478°C pour former le flux (3). Le chauffage peut être réalisé par un échangeur (H) dont le fluide chaud est constitué des fumées de l'oxydeur thermique (J).

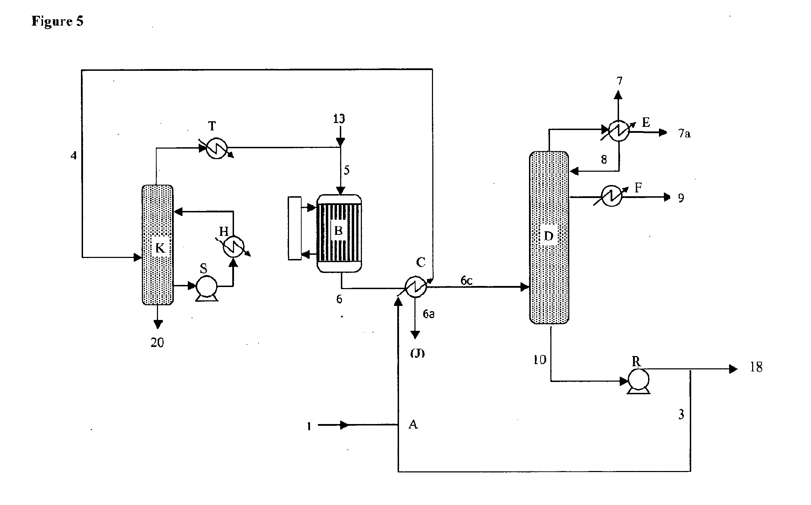

**EXEMPLE 2 (en référence à la figure 5) :** Déshydratation en phase gaz du glycérol pour produire de l'acroléine comprenant une séparation de produits lourds par flash juste avant l'étape de déshydratation du glycérol et une séparation de produits lourds par condensation juste après l'étape de déshydratation de glycérol.

Un flux liquide (1) de glycérol à 25°C (210 kg/h, 99,0% glycérol) et un flux liquide recyclé (3) (519 kg/h, 124°C, 93% eau, 2,6% d'acide acétique, 2,1% d'acide acrylique et 1,0% d'hydroxypropanone) sont mélangés en (A) puis préchauffés à 212°C sous 25 bars via les échangeurs (C) puis envoyés dans une colonne de flash (K) fonctionnant sous 2,8 bar absolu qui permet de séparer les sous-produits lourds. Cette colonne comporte une boucle sur la phase liquide munie d'une pompe (S) et d'un échangeur de chaleur (H) destiné à apporter les calories nécessaires à la vaporisation du glycérol et de l'eau. On récupère en pied de la colonne (K) un flux de sous-produits lourds (20) de 3 kg/h à 244°C qui sont éliminés. La phase gaz sortant en tête de cette colonne (K) est envoyée dans un échangeur (T) pour la réchauffer à 303°C, puis est mélangée à un flux d'oxygène (13) (14 kg/h) pour former le flux (5) (740 kg/h, 2,8 bar absolu, 300°C, 65% eau, 28% glycérol, 1,9% oxygène, 1,8% acide acétique, 1,5% acide acrylique).

Le flux gazeux (5) est envoyé dans un réacteur multitubulaire à lit fixe (B) contenant un catalyseur hétérogène acide de déshydratation et couplé à un bain de sel fondu qui permet d'évacuer la chaleur produite par les réactions. De ce réacteur, sort un flux gazeux (6) à 314°C (76% eau, 13,6% acroléine, 2,1% acide acétique, 1,7% acide acrylique, 1,2% acétaldéhyde, 1,0% de monoxyde de carbone).

Ce flux est refroidi à 123°C dans les échangeurs de chaleur (C) déjà décrits, qui permettent de récupérer un flux liquide (6a) de produits lourds (29 kg/h) et une fraction gazeuse (6c). Le flux liquide est envoyé vers un oxydeur thermique (J). La fraction gazeuse est envoyée dans une colonne d'absorption (D). La colonne (D) comporte en tête un condenseur (E). On récupère en tête une phase gaz (7) (26 kg/h, 0°C, 2,3 bar, 29% CO, 22% $CO_2$, 19% acétaldéhyde, 18% formaldéhyde, 13% $O_2$) et une phase liquide en sortie du condenseur (E) qui est partiellement soutirée (2 kg/h, 0°C, 2,3 bar, 78% acétaldéhyde, 15% formaldéhyde, 4% acroléine) et partiellement recyclée dans la colonne. La colonne comporte un soutirage latéral d'un flux qui est condensé au moyen de l'échangeur (F) pour donner un flux (9) d'acroléine (109 kg/h, 30°C, 92,2% acroléine, 4,9% eau, 2,2% acétaldéhyde).

Le flux liquide (10) appauvri en acroléine sortant en pied de la colonne (D) (574 kg/h, 124°C, 93% d'eau, <0,1% d'acroléine, 2,6% d'acide acétique, 2,1% d’acide acrylique, 1,0% d'hydroxypropanone) est partagé en un flux 18 qui est éliminé (55 kg/h) et le flux (3) décrit plus haut.

**EXEMPLE 3 :** Essai laboratoire selon l'invention

Un catalyseur acide de déshydratation est préparé par imprégnation au volume poreux d'une solution aqueuse d'acide phosphotungstique sur un oxyde de titane réduit à la granulométrie de 300-500 $\mu$m. Le catalyseur est séché à l'étuve ventilée à 110°C puis calciné pendant 3 heures à 500°C. Un volume de 7 ml du catalyseur est introduit dans un réacteur en inox 316L de diamètre 13 mm placé verticalement dans un four chauffé à 280°C.

Un débit de 15 g/h d'une solution constituée de 50% massique de glycérol pur et 50% d'eau est mélangé avec un débit d'oxygène et d'azote de respectivement 1,2 et 18 normaux litres / heure, puis envoyé dans un vaporiseur qui chauffe le mélange à 280°C et qui est connecté au réacteur.

Les effluents gazeux en sortie du réacteur sont soit envoyés vers deux pièges en série contenant initialement 120 et 80 grammes d'eau et refroidis à 0°C de façon à piéger totalement l'acroléine pour faire un bilan matière, soit envoyés vers un réservoir refroidi à 0°C de façon à piéger la majeure partie de l'eau et des lourds produits par la réaction. Dans les deux cas, les gaz non condensés sont éliminés à l'atmosphère après lavage dans un piège contenant une solution diluée de soude.

On mesure la perte de charge du réacteur au long de l'expérience.

On a effectué un bilan matière avec les pièges en série remplis d'eau du temps t = 2 heures à t = 3 heures 30 d'une part et du temps t = 21 heures à t = 22 heures 30. On a mesuré par chromatographie gaz la teneur en glycérol et en acroléine dans les pièges. On calcule la conversion en glycérol et le rendement en acroléine selon les formules :

$$\text{Conversion glycérol (\%)} = (\text{(moles de glycérol injectées dans le réacteur pendant la durée du bilan)} - \text{(moles de glycérol récupérées dans les 2 pièges)}) / \text{(moles de glycérol injectées dans le réacteur pendant la durée du bilan)} * 100$$

$$\text{Rendement acroléine (\%)} = \text{(moles d'acroléine récupérée dans les 2 pièges)} / \text{(moles de glycérol injectées dans le réacteur pendant la durée du bilan)} * 100$$

Les résultats de l'expérience sont reportés au tableau 1 ci-dessous.

Par ailleurs, on a collecté dans le réservoir la majeure partie de l'eau et des lourds entre le temps t = 1 heure et le temps t = 2 heures, ainsi qu'entre le temps t = 3 heures 30 et le temps t = 21 heures. Cette solution aqueuse a ensuite été traitée pendant 2 heures à l'évaporateur rotatif chauffé à 30 °C et sous vide partiel, de façon à évaporer l'acroléine. La collecte dans le réservoir et l'évaporation permettent de simuler l'étape (D) représentée à la figure 1. Afin de simuler l'étape (G) de la figure 1, la solution aqueuse débarrassée de l'acroléine est distillée à l'évaporateur rotatif sous vide partiel à 75°C, de façon à récupérer un condensat d'eau recyclée d'une part et un résidu de produit organiques très colorés dans le distillat d'autre part.

Afin de simuler les étapes (A) et (B) de la figure 1, on a mélangé le condensat d'eau recyclée avec du glycérol de façon à obtenir une solution à 50% de glycérol et on a renouvelé l'expérience de déshydratation du glycérol. Les résultats sont reportés au tableau 1.

Lorsqu'on utilise de l'eau recyclée qui a subi une séparation des lourds par distillation, on obtient des conversions du glycérol et des rendements en acroléine similaires à ceux que l'on a en utilisant de l'eau pure.

**EXEMPLE 4 :** Comparatif

De la même façon qu'à l'exemple 3, on a réalisé la réaction de déshydratation du glycérol avec une solution constituée de 50% massique de glycérol pur et 50% d'eau et on a collecté dans un réservoir la majeure partie de l'eau et des lourds, qui ont été traités pendant 2 heures à l'évaporateur rotatif chauffé à 30 °C et sous vide partiel, de façon à évaporer l'acroléine.

On a alors mélangé directement la solution aqueuse récupérée contenant un mélange eau et lourds recyclés avec du glycérol pur pour préparer une solution à 50% de glycérol et on a renouvelé l'expérience de déshydratation du glycérol. Pendant 3 heures 30, on a observé une perte de charge de 0,1 bar sur le réacteur, puis la pression a augmenté progressivement pour atteindre 0,3 bar après 5 heures et dépasser 1 bar après 7 heures. L'expérience n'a pas pu être poursuivie.

Les résultats sont reportés au tableau 1.

Lorsqu'on utilise de l'eau recyclée contenant des lourds, on a observé une montée très rapide en pression consécutive à un bouchage du réacteur.

**Tableau 1**

| | Conversion glycérol après 2h / 21 h (%) | Rendement acroléine après 2h / 21 h (%) | Perte de charge après 2h / 5h / 21h (bar) |
|---|---|---|---|
| Exemple 3 Glycérol / eau | >99 / 80 | 70 / 48 | 0,1 / 0,1 / 0,1 |
| Exemple 3 Glycérol / condensat eau recyclée | >99 / 79 | 70 / 47 | 0,1 / 0,1 / 0,1 |
| Exemple 4 comparatif Glycérol / eau et lourds recyclés sans étape de distillation | >99 / non mesurable | 70 / non mesurable | 0,1 /0,3 / >1 (7h) |

**Revendications**

1. Procédé de fabrication d'acroléine à partir de glycérol comprenant au moins les étapes suivantes :

   a) on soumet du glycérol à une réaction de déshydratation pour obtenir un flux aqueux contenant de l'acroléine,
   b) on sépare le flux issu de l'étape a) en une phase riche en acroléine et une phase aqueuse appauvrie en acroléine, dans des conditions telles que 20% à 95% massiques de l'eau présente dans le flux issu de l'étape a) sont éliminés dans la phase appauvrie en acroléine,
   c) on recycle tout ou partie de la phase aqueuse appauvrie en acroléine à l'étape a), **caractérisé en ce que** l'on met en oeuvre une étape de séparation de composés organiques plus lourds que l'eau par différence de point d'ébullition, appliquée à l'un au moins des flux suivants : le flux entrant à l'étape a), le flux aqueux sortant de l'étape a), la phase aqueuse appauvrie en acroléine de l'étape b).

2. Procédé selon la revendication 1 comprenant en outre un traitement de purification de ladite phase riche en acroléine par absorption/distillation.

3. Procédé de fabrication d'acide acrylique à partir de glycérol comprenant au moins les étapes suivantes :
   on produit de l'acroléine selon la revendication 1,

   d) on soumet la phase riche en acroléine à une réaction d'oxydation catalytique pour obtenir un flux contenant de l'acide acrylique,
   e) on soumet le flux issu de l'étape d) à un ou plusieurs traitements de purification, et l'on récupère l'acide acrylique purifié.

4. Procédé selon l'une quelconque des revendications 1 à 3 **caractérisé en ce que** l'on applique une étape de séparation des composés organiques plus lourds que l'eau par différence de point d'ébullition au flux entrant à l'étape a) et/ou à la phase aqueuse appauvrie en acroléine de l'étape b), associée ou non à une étape de séparation de composés organiques plus lourds que l'eau par différence de point d'ébullition appliquée au flux aqueux sortant de l'étape a).

5. Procédé selon l'une quelconque des revendications précédentes **caractérisé en ce que** l'étape de séparation des composés organiques plus lourds que l'eau est réalisée, soit par évaporation lorsque le flux à séparer est initialement liquide, soit par condensation lorsque le flux à séparer est initialement gazeux, soit par une combinaison d'évaporation et de condensation lorsqu'on utilise plusieurs étages de séparation.

6. Procédé selon l'une quelconque des revendications précédentes **caractérisé en ce que** l'étape d'élimination des composés organiques plus lourds que l'eau est réalisée par évaporation du mélange de glycérol (1) et de la phase riche en eau recyclée (3), dans un appareil (K), qui génère une phase riche en glycérol et en eau et appauvrie en composés organiques plus lourds que l'eau (flux (5)), qui est envoyée dans le réacteur de déshydratation (B), et une phase (20) liquide ou solide riche en composés organiques plus lourds que l'eau qui est éliminée.

7. Procédé selon l'une quelconque des revendications 1 à 5 **caractérisé en ce que** l'étape d'élimination des composés organiques plus lourds que l'eau est réalisée par un traitement du flux (6) sortant du réacteur de déshydratation (B), dans un appareil (C), qui génère une phase gaz (6c) appauvrie en composés organiques plus lourds que l'eau et une phase (6a) liquide ou solide enrichie en composés organiques plus lourds que l'eau qui est éliminée.

8. Procédé selon l'une quelconque des revendications 1 à 5 **caractérisé en ce que** l'étape d'élimination des composés organiques plus lourds que l'eau est réalisée par évaporation du flux (10) sortant de l'unité de séparation (D), dans un appareil (G), qui génère une phase gaz (3) riche en eau qui est recyclée, et une phase (18) liquide ou solide enrichie en composés organiques plus lourds que l'eau qui est éliminée.

9. Procédé selon l'une quelconque des revendications précédentes **caractérisé en ce que** la phase aqueuse recyclée à l'étape de déshydratation a) sous forme de flux gazeux (3) est utilisée pour vaporiser le flux de glycérol dans une étape de mélange (A) avant d'envoyer le flux gazeux réactif dans le réacteur de déshydratation (B).

10. Procédé selon la revendication 9 **caractérisé en ce que** le flux de glycérol est injecté dans la chambre de mélange (A) via des buses de pulvérisation ou d'atomisation.

11. Procédé selon l'une quelconque des revendications précédentes **caractérisé en ce que** l'étape a) est réalisée en phase gaz et on utilise une pompe à chaleur pour récupérer l'énergie de condensation de l'eau du flux réactionnel gazeux à l'issue de l'étape a) et pour vaporiser la phase aqueuse appauvrie en acroléine séparée de la phase enrichie en acroléine lors de l'étape b).

12. Procédé selon l'une quelconque des revendications précédentes **caractérisé en ce que** l'étape a) est réalisée en phase gaz et on comprime le flux gazeux issu de l'étape a) afin de pouvoir récupérer l'énergie de condensation de l'eau du flux réactionnel lors de l'étape b).

**Patentansprüche**

1. Verfahren zur Herstellung von Acrolein aus Glycerin, das mindestens die folgenden Schritte umfasst:

   a) man unterwirft das Glycerin einer Dehydratisierungsreaktion, wobei man einen Acrolein enthaltenden wässrigen Strom erhält,
   b) man trennt den Strom aus Schritt a) in eine acroleinreiche Phase und eine an Acrolein abgereicherte wässrige Phase, und zwar unter solchen Bedingungen, dass 20 bis 95 Gew.-% des in dem Strom aus Schritt a) vorliegenden Wassers in der an Acrolein abgereicherten wässrigen Phase eliminiert werden,
   c) man führt die gesamte an Acrolein abgereicherte wässrige Phase oder einen Teil davon in Schritt a) zurück, **dadurch gekennzeichnet, dass** man einen Schritt der Abtrennung von organischen Verbindungen, die schwerer als Wasser sind, durch Siedepunktsdifferenz durchführt, der auf mindestens einen der folgenden Ströme angewendet wird: den in Schritt a) eintretenden Strom, den aus Schritt a) austretenden wässrigen Strom, die an Acrolein abgereicherte wässrige Phase aus Schritt b).

2. Verfahren nach Anspruch 1, das außerdem eine Reinigungsbehandlung der acroleinreichen Phase durch Absorption/Destillation umfasst.

3. Verfahren zur Herstellung von Acrylsäure aus Glycerin, das mindestens die folgenden Schritte umfasst:

   man stellt gemäß Anspruch 1 Acrolein her,

      d) man unterwirft die acroleinreiche Phase einer katalytischen Oxidationsreaktion, wobei man einen Acrylsäure enthaltenden Strom erhält,

e) man unterwirft den Strom aus Schritt d) einer oder mehreren Reinigungsbehandlungen und gewinnt die gereinigte Acrylsäure.

4. Verfahren nach einem der Ansprüche 1 bis 3, **dadurch gekennzeichnet, dass** man einen Schritt der Abtrennung der organischen Verbindungen, die schwerer als Wasser sind, durch Siedepunktsdifferenz auf den in Schritt a) eintretenden Strom und/oder die an Acrolein abgereicherte wässrige Phase aus Schritt b) anwendet, gegebenenfalls in Kombination mit einem auf den aus Schritt a) auftretenden wässrigen Strom angewendeten Schritt der Abtrennung der organischen Verbindungen, die schwerer als Wasser sind, durch Siedepunktsdifferenz.

5. Verfahren nach einem der vorhergehenden Ansprüche, **dadurch gekennzeichnet, dass** der Schritt der Abtrennung der organischen Verbindungen, die schwerer als Wasser sind, entweder durch Verdampfung, wenn der zu trennende Strom anfangs flüssig ist, oder durch Kondensation, wenn der zu trennende Strom anfangs gasförmig ist, oder durch eine Kombination von Verdampfung und Kondensation bei Verwendung mehrerer Trennschritte durchgeführt wird.

6. Verfahren nach einem der vorhergehenden Ansprüche, **dadurch gekennzeichnet, dass** der Schritt der Abtrennung der organischen Verbindungen, die schwerer als Wasser sind, durch Verdampfung des Gemischs von Glycerin (1) und der zurückgeführten wasserreichen Phase (3) in einer Apparatur (K) durchgeführt wird, welche eine glycerin- und wasserreiche und an organischen Verbindungen, die schwerer als Wasser sind, abgereicherte Phase (Strom (5)), die in den Dehydratisierungsreaktor (B) eingeleitet wird, und eine an organischen Verbindungen, die schwerer als Wasser sind, reiche flüssige oder feste Phase (20), die eliminiert wird, erzeugt.

7. Verfahren nach einem der Ansprüche 1 bis 5, **dadurch gekennzeichnet, dass** der Schritt der Abtrennung der organischen Verbindungen, die schwerer als Wasser sind, durch Behandlung des aus dem Dehydratisierungsreaktor (B) austretenden Stroms (6) in einer Apparatur (C) durchgeführt wird, welche eine an organischen Verbindungen, die schwerer als Wasser sind, abgereicherte Gasphase (6c) und eine an organischen Verbindungen, die schwerer als Wasser sind, reiche flüssige oder feste Phase (6a), die eliminiert wird, erzeugt.

8. Verfahren nach einem der Ansprüche 1 bis 5, **dadurch gekennzeichnet, dass** der Schritt der Abtrennung der organischen Verbindungen, die schwerer als Wasser sind, durch Verdampfung des aus der Trenneinheit (D) austretenden Stroms (10) in einer Apparatur (G) durchgeführt wird, welche eine wasserreiche Gasphase (3), die zurückgeführt wird, und eine an organischen Verbindungen, die schwerer als Wasser sind, reiche flüssige oder feste Phase (18), die eliminiert wird, erzeugt.

9. Verfahren nach einem der vorhergehenden Ansprüche, **dadurch gekennzeichnet, dass** die in Form des Gasstroms (3) in den Dehydratisierungsschritt a) zurückgeführte wässrige Phase zum Verdampfen des Glycerinstroms in einem Mischschritt (A) vor dem Einleiten des reaktiven Gasstroms in den Dehydratisierungsreaktor (B) verwendet wird.

10. Verfahren nach Anspruch 9, **dadurch gekennzeichnet, dass** der Glycerinstrom über Sprüh- oder Zerstäubungsdüsen in die Mischkammer (A) eingeleitet wird.

11. Verfahren nach einem der vorhergehenden Ansprüche, **dadurch gekennzeichnet, dass** der Schritt a) in der Gasphase durchgeführt wird und man eine Wärmepumpe zur Rückgewinnung der Energie der Kondensation des Wassers des gasförmigen Reaktionsstroms aus Schritt a) und zur Verdampfung der von der acroleinreichen Phase abgetrennten, an Acrolein abgereicherten wässrigen Phase während Schritt b) verwendet.

12. Verfahren nach einem der vorhergehenden Ansprüche, **dadurch gekennzeichnet, dass** der Schritt a) in der Gasphase durchgeführt wird und man den Gasstrom aus Schritt a) komprimiert, um die Energie der Kondensation des Wassers des Reaktionsstroms während Schritt b) zurückgewinnen zu können.

## Claims

1. Process for the manufacture of acrolein from glycerol comprising at least the following stages:

a) glycerol is subjected to a dehydration reaction in order to obtain an aqueous stream comprising acrolein,
b) the stream resulting from stage a) is separated into an acrolein-rich phase and an acrolein-poor aqueous phase, under conditions such that from 20% to 95% by weight of the water present in the stream resulting from stage a) are removed in the acrolein-poor phase,

c) all or part of the acrolein-poor aqueous phase is recycled to stage a),

**characterized in that** a stage of separation of organic compounds heavier than water by difference in boiling point is carried out, which stage is applied to one at least of the following streams: the stream entering stage a), the aqueous stream exiting from stage a) or the acrolein-poor aqueous phase of stage b).

2. Process according to Claim 1, additionally comprising a purification treatment of the said acrolein-rich phase by absorption/distillation.

3. Process for the manufacture of acrylic acid from glycerol comprising at least the following stages:
acrolein is produced according to Claim 1,

d) the acrolein-rich phase is subjected to a catalytic oxidation reaction in order to obtain a stream comprising acrylic acid,

e) the stream resulting from stage d) is subjected to one or more purification treatments and purified acrylic acid is recovered.

4. Process according to any one of Claims 1 to 3, **characterized in that** a stage of separation of the organic compounds heavier than water by difference in boiling point is applied to the stream entering stage a) and/or to the acrolein-poor aqueous phase of stage b), in combination or not in combination with a stage of separation of organic compounds heavier than water by difference in boiling point applied to the aqueous stream exiting from stage a).

5. Process according to any one of the preceding claims, **characterized in that** the stage of separation of the organic compounds heavier than water is carried out either by evaporation, when the stream to be separated is initially liquid, or by condensation, when the stream to be separated is initially gaseous, or by a combination of evaporation and of condensation, when several separation stages are used.

6. Process according to any one of the preceding claims, **characterized in that** the stage of removal of the organic compounds heavier than water is carried out by evaporation of the mixture of glycerol (1) and of the recycled water-rich phase (3) in a device (K), which generates a phase rich in glycerol and in water and depleted in organic compounds heavier than water (stream (5)), which is conveyed to the dehydration reactor (B), and a liquid or solid phase (20) rich in organic compounds heavier than water, which is eliminated.

7. Process according to any one of Claims 1 to 5, **characterized in that** the stage of removal of the organic compounds heavier than water is carried out by a treatment of the stream (6) exiting from the dehydration reactor (B) in a device (C), which generates a gas phase (6c) depleted in organic compounds heavier than water and a liquid or solid phase (6a) enriched in organic compounds heavier than water, which is eliminated.

8. Process according to any one of Claims 1 to 5, **characterized in that** the stage of removal of the organic compounds heavier than water is carried out by evaporation of the stream (10) exiting from the separating unit (D) in a device (G), which generates a water-rich gas phase (3), which is recycled, and a liquid or solid phase (18) enriched in organic compounds heavier than water, which is eliminated.

9. Process according to any one of the preceding claims, **characterized in that** the aqueous phase recycled to the dehydration stage a) in the form of the gas stream (3) is used to evaporate the glycerol stream in the mixing stage (A) before conveying the reactive gas stream to the dehydration reactor (B).

10. Process according to Claim 9, **characterized in that** the glycerol stream is injected into the mixing chamber (A) via spray or atomization nozzles.

11. Process according to any one of the preceding claims, **characterized in that** stage a) is carried out in the gas phase and a heat pump is used to recover the energy of condensation of the water of the gaseous reaction stream on conclusion of stage a) and to evaporate the aqueous phase depleted in acrolein separated from the phase enriched in acrolein during stage b).

12. Process according to any one of the preceding claims, **characterized in that** stage a) is carried out in the gas phase and the gas stream resulting from stage a) is compressed in order to be able to recover the energy of condensation of the water of the reaction stream during stage b).

Figure 1

Figure 2

Figure 3

Figure 4

Figure 5

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2006092271 A
- FR 695931
- US 2558520 A
- WO 9905085 A
- US 5387720 A
- WO 06087083 A
- WO 06087084 A
- WO 09044081 A
- EP 1710227 A
- WO 06136336 A
- WO 2006092272 A
- WO 08087315 A
- EP 1978009 A
- WO 06114506 A
- US 2008018319 A
- WO 2006136336 A
- US 3433840 A
- EP 1300384 A
- EP 1474374 A
- EP 995491 A
- EP 1147807 A
- US 20050020851 A
- FR 2146386
- US 5426221 A
- FR 9614397
- WO 1031949 A
- FR 0955111
- FR 0955112
- FR 1051961
- FR 2928648

**Littérature non-brevet citée dans la description**

- **K. TANABE et al.** Studies in Surface Science and Catalysis. 1989, vol. 51
- *Techniques de l'Ingénieur, Traité des Procédés,* vol. J 6 100, 1-4